# EUROPEAN PATENT APPLICATION

(11) **EP 2 014 267 A1**
(43) Date of publication of application: **14.01.2009**
(21) Application number: 07110402.0
(22) Date of filing: 15.06.2007
(51) Int. Cl.: A61F 13/15, A61F 13/42, G08B 23/00

(54) **Universal wet garment detector**

(71) Applicant: Intensia NV, 9100 Sint-Niklaas (BE)
(72) Inventor: Herbots, Herman, 3370, Boutersem (BE)
(74) Representative: Plas, Axel Ivo Michel

(57) **Abstract**

A wet garment detector (101) for detecting a wetness condition of garment comprises a housing (103) for containing electronic components, a wetness sensor (201) at least partially contained in said housing (103), and means (104, 105) for piercing the detector (101) onto said garment to thereby removably attach the detector (101) to the garment.

## Description

### Field of the Invention

The present invention generally relates to the detection of the wetness status or wetness condition of garment, e.g. a diaper or undergarment, in order to enable a relative or care-giver to exchange the diaper of an incontinent person, baby or child within a tolerable time limit in order to avoid full saturation of the garment and leakage resulting in wet clothes, sheets, etc. In particular, the current invention relates to a universal, reusable wetness detector able to detect a certain degree of saturation of the garment that is being monitored.

### Background of the Invention

The detection of wetness in diapers or undergarment and automated signalling thereof, for instance through an audible signal or a communication signal sent to a receiver, enables care-givers and relatives to timely change the diaper or undergarment of an incontinent person (or baby or child) without regularly uncovering the sleeping incontinent person. Since the incontinent person need no longer be awakened and checked for loss of fluid at regular intervals, the comfort of both the incontinent patient and the care-giver is significantly increased through wet garment detectors. Further, wetness detection and automated signalling thereof enables the use of smaller (and thus cheaper) diapers, since the need for spare capacity in the diapers to absorb larger fluid volumes freed in between two rounds of checking reduces. Also, medical problems like skin allergies resulting from being in touch with a wet diaper for a relatively long time period are avoidable through the use of wet garment detectors that enable to change the diaper instantly whenever a wetness condition is flagged. Several prior art solutions exist for wet garment detection. The most relevant ones in view of the present invention are described in the following paragraphs.

A wet diaper informing device reporting to a care-giver the number of times a diaper gets wet or the time elapsed since the diaper got wet such that the care-giver can determine whether delaying the exchange of the diaper is tolerable, is known for instance from Japanese patent application no. JP2005000602, entitled "Wet Diaper Informing and Indicating Device and Diaper Equipped with Wet Detecting Device". This prior art detector is integrated in the diaper and has a wetness sensor, an RFID tag, an integrated circuit (IC) with storage capacity, and a printed antenna for communication with a display device that is held by the care-giver. Since the wet diaper detector is integrated in the diaper, the detector is not reusable, and the user or patient is restricted to buy and use a particular type of diaper from a particular manufacturer. Further, the detector is rather complex, with integrated RFID technology, printed antenna and integrated memory for storing the number of times that wet signals are generated, rendering the detector and consequently also the diaper wherein the detector is integrated rather expensive. Further, each care-giver needs to be equipped with the accompanying proprietary display device.

Alternative wet garment detectors, such as the one described in United States patent US 7,049,969, are not integrated in the diaper to be monitored but require the diaper to be furnished with a little pocket for holding the moisture detector or with a belt whereto the moisture detector can be attached through a clips. The liquid detector of US 7,049,969 further has a capacitive sensor and a housing with through-hole enabling the liquid to infiltrate in the dielectric of the capacitive sensor. A resonant circuit and radio-wave emitter produce the signal indicative for the wetness condition of the diaper. Although such solution has the advantage over the detector known from JP2005000602 to be reusable, it still requires dedicated diapers or incontinence garment featuring a pocket or belt. Further, since the fluids infiltrate the housing, washing the detector to be reused with a different patient, e.g. in a hospital, is rather difficult.

The wet garment detector described in United States patent US 5,903,222 consists of a capacitive sensor located within a housing that can be affixed to the exterior surface of the garment being monitored for wetness. To affix the detector to the exterior of the garment, clips, adhesive patches, or preformed pockets in the garment may be used, as is indicated in column 6, lines 61-67 of US 5,903,222. The wetness detector may have an audible alarm such as a speaker or buzzer, or a transmitter for signalling the wetness condition to a remote receiver. In applications where multiple detectors are employed, e.g. a hospital or nursing home, each detector may be identifiable through an encoded unique address that is transmitted together with the wetness indication signal. Although the prior art wet garment detector of US 5,903,222 is reusable and intended for use with undergarments of any type, e.g. disposable paper diapers or cloth diapers, the disclosed embodiments either require the use of dedicated garment, e.g. diapers having a pre-formed external pocket, or alternatively make use of means which are rather difficult to become securely affixed to the exterior surface of diapers, such as clips or adhesive pouches, unless the latter diapers are pre-formed to receive such clips or adhesive pouches. In addition, US 5,903,222 does not teach selecting the saturation level whereat the wetness condition is signalled.

Equivalently to US 5,903,222, PCT Patent Application WO 95/15739 describes a moisture detector which is either integral part of the pants or alternatively may be located in the crotch of the pants and be removable to become reusable. The detector contains an electric sensor based on two copper strips, and a light-emitting diode (LED) to visibly indicate a wetness condition, an audible alarm such as a piezoelectric element, or a transmitter for indication of the wetness condition to a remote receiver. Again, pockets, pouches and conventional fixing means are suggested for keeping the detector in the crotch of the pants, requiring dedicated garment and/or clips or pouches that are difficult to become securely attached to the crotch of diapers. WO 95/15739 does not disclose selecting the wetness level or saturation degree whereat signalling takes place.

Still another prior art solution, described in PCT Patent Application WO 96/14813 describes applications wherein plural wetness detectors corresponding to multiple patients, are monitored by a single incontinence management system which further connects to a paging system to call staffing assistance. The wetness detectors contain battery based electronic moisture sensors and transmitters that send coded radio signals to a receiver in a device held by the care-giver or in a central station.

It is an object of the present invention to disclose a wet garment detector which overcomes the above mentioned drawbacks and shortcomings of the prior art detectors. In particular, it is an objective to disclose a universal wet garment detector, i.e. a wet garment detector that can be used with any type of garment of any manufacturer, which does not require adaptation of the garment like for instance the presence of pockets, and which does not rely on fixing means such as a clips that cannot be securely attached to the garment without using belts or specific pre-formed garment. It is a further objective to disclose a reusable, washable wet garment detector. Further, it is an objective to provide a detector that enables selection of the wetness level or saturation degree of the garment whereat the wetness condition is signalled.

### Summary of the Invention

According to the present invention, the above objectives are realized and the shortcomings of the prior art solutions are overcome by the wet garment detector defined by claim 1, comprising:
a housing for containing electronic components;
a wetness sensor at least partially contained in the housing; and
means for piercing the detector onto the garment to thereby removably attach the detector to the garment.

Indeed, piercing the detector into the garment securely attaches the detector to any type of garment and can be realized with means as simple as a single or plural pins that extend to the exterior of the housing. The pierced detector can be removed from the garment by simply pulling it away, thus enabling to reuse the detector with the same or a different patient. By selecting the distance from the location where the detector is pierced into the garment to the crotch of the garment, the saturation level whereat the wetness condition is signalled becomes selectable in a straightforward, dummy-proof manner. Advantageously, the detector according to the present invention may be used in conjunction with garment that contains visible indications for the saturation level, e.g. stripes or markings on the exterior surface of a diaper indicative for the saturation level of the diaper that is sensed when the detector according to the present invention is pierced at the marked position.

In addition to the wet garment detector defined in claim 1, the current invention relates to a corresponding method for detecting a wetness condition of garment as defined by claim 10, the method comprising removably attaching a wet garment detector having a housing and wetness sensor at least partially contained in the housing to the garment by piercing the wet garment detector onto the garment.

In a preferred but optional embodiment of the wet garment detector according to the present invention, the means for piercing comprise two detection electrodes that form part of the wetness sensor. This preferred embodiment is defined by claim 2.

Thus, although the skilled person will appreciate that variant implementations of the detector may comprise a single or multiple pins or needles that allow to attachably pierce the detector onto the garment, an embodiment where the detector is pierced into the garment using electrodes of the wetness sensor is advantageous in that no additional hardware is needed to pierce the detector onto the garment in case the sensor is an electronic wetness sensor having two wetness electrodes since the electrodes themselves can be used for the piercing.

Optionally, the wet garment detector according to the present invention has detection electrodes that are composed of non-corroding material, as defined by claim 3.

Indeed, in particular in an embodiment of the present invention where the electrodes of the wetness sensor are used to pierce the incontinence garment, and where these electrodes get in direct contact with the fluids, it is important to manufacture the electrodes out of non-corroding materials such as for instance stainless steel.

Further optionally, the detection electrodes of a preferred embodiment of the wet garment detector according to the present invention are U-shaped, one leg of each U-shaped detection electrode extending outside the housing for piercing into the garment and a second leg of each U-shaped detection electrode extending inside the housing for connectivity to electronic components of the wetness sensor.

U-shaped detection electrodes enable to sense wetness of the garment at a location in the garment that is below the location where the electrodes are pierced through the plastic or fluid-tight exterior surface of the garment. This way, one avoids that fluids are leaking outside the garment via the piercing holes in the exterior surface of the garment before the wetness condition is detected and signalled. An additional advantage of the U-shaped detection electrodes is that such shape more securely affixes the detector to the garment than for instance straight, linear detection electrodes would do. Yet another advantage of U-shaped electrodes is that movements by the person wearing the garment wherein the detector according to the invention is pierced, e.g. while sleeping, will not result in the detector sliding out of the garment since the U-shaped electrodes shall keep the detector at its position once pierced into the garment.

As is indicated by claim 5, the wetness sensor in an optional embodiment of the detector according to the present invention may comprise:
two detection electrodes adapted to be non-conductive in a first state wherein the wetness condition is not detected, and to be conductive in a second state wherein the wetness condition is detected;
a power supply, having a first terminal permanently connected to a first power supply terminal of an active element able to signal the wetness condition, and having a second terminal;
a switch whose control terminal is connected to a detection electrode of the detection electrodes, and which is adapted to connect the second terminal of the power supply to a second power supply terminal of the active element when the detection electrodes become conductive.

This way, a relatively simple-to-implement electronic wetness sensor is realized which conducts when a wetness condition is detected and which remains non-conductive as long as the garment stays dry. When conducting, the positive and negative poles of the power supply, e.g. a battery, a rechargeable battery, the AC net power supply or a down-converted DC voltage are connected to the unit that signals the wetness condition by the switch. Since the electrodes are only conductive when they become wet, the power consumption of such electronic wetness sensor is relatively low. The current can for instance be restricted to 30 µA (micro-Ampères). Depending on the complexity of the active element, e.g. a wired or wireless transmitter, the power consumption will increase during the signalling but should still enable 2 to 3 years of functioning without replacement of the batteries. As an alternative to an electronic resistive-based sensor, the wetness sensor can be capacitive, inductive, based on a resonant-circuit, a chemical or electro-chemical wetness sensor, etc.

Further optionally, the wet garment detector according to the present invention may comprise means for visibly or audibly signalling the wetness condition of the garment, as defined by claim 6.

Means for audibly or visibly signalling the wetness condition comprise but are not limited to a loudspeaker, a buzzer, a ringer, a light-emitting diode (LED), a liquid crystal display (LCD) screen, etc.

Alternatively or complementarily, as indicated by claim 7, the wet garment detector according to the present invention may comprise a transmitter for wired or wireless transmission of a wetness signal to a receiver.

Thus, the detector may include a transmitter that sends wetness signals to a remote receiver, e.g. contained in a handheld device like a mobile phone, a personal digital assistant (PDA), a laptop or a dedicated receiver carried by the care-giver, to a patient terminal present in the room of every patient and integrated for instance with building automation control functions for the patient's room and/or other patient monitoring functions, or to a central unit such as a server in a local area network (LAN), a paging system, an alarm monitoring platform in a nursing home, etc. The connection between the transmitter in the detector according to the present invention and the receiver may be wired or wireless, and may use standardized or proprietary communication protocols.

Still optionally, the receiver in the wet garment detector according to the present invention may be adapted to also receive signalling from an alarm generating device, a medical sensor, etc. This optional feature of the wet garment detector according to the present invention is defined by claim 8.

This way, the receiver, e.g. embedded in a patient terminal or a handheld device, may not only receive signals indicative for the wetness condition of the patient's diaper, but also be able to receive and interpret signals from other sensors, e.g. a blood pressure sensor, a heart pulse sensor, etc. and/or signals from an alarm button near the patient's bed.

Yet another option of the wet garment detector according to the present invention, is that the means for piercing are adapted to be pierced at different locations onto the garment, to thereby enable selection of the wetness condition of the garment detectable by the detector. This option is covered by claim 9.

Indeed, as already indicated above, the saturation degree or wetness level of the diaper or garment whereat the wetness condition is signalled, may be selected via the location on the exterior surface of the garment where the detector is pierced. Advantageously, the piercable detector according to the present invention may even be used in conjunction with diapers or garment that have visual marks on their exterior surface indicative for the saturation level that is sensed when the detector is pierced at the location marked.

In line with claim 9, an embodiment of the method according to the present invention defined by claim 11 further comprises determining a location onto the garment thereby selecting the wetness condition of the garment detectable by the detector, and piercing the wet garment detector at that location onto the garment.

### Brief Description of the Drawings

Fig. 1 illustrates an embodiment of the wet garment detector 101 according to the present invention;
Fig. 2 illustrates in more detail the electronic circuitry of the wetness sensor 201 that forms part of the wet garment detector 101 drawn in Fig. 1;
Fig. 3 illustrates the use of the wet garment detector 101 of Fig. 1 in a nursery home or hospital; and
Fig. 4 shows the external surface of incontinence garment 401 which is advantageously used in conjunction with the wet garment detector 101 according to the present invention.

### Detailed Description of Embodiment(s)

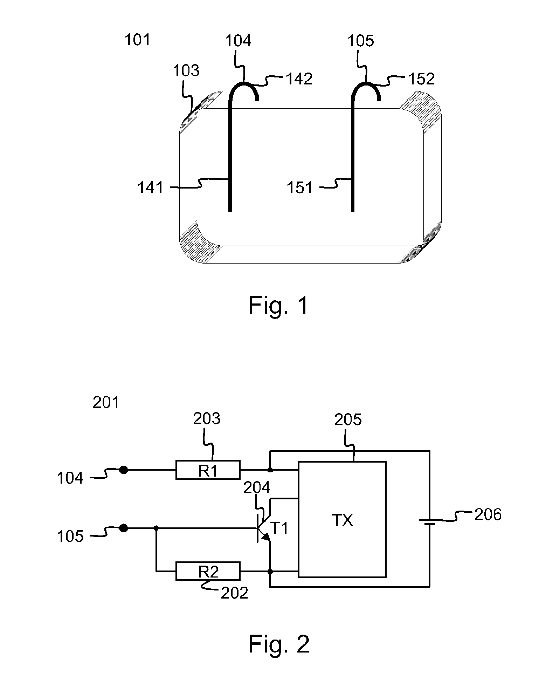

The wet garment detector 101 drawn in Fig. 1 has a housing 103 that contains the electronic circuitry of a resistive wetness sensor and a wireless transmitter. The housing 103 has two openings through which two U-shaped electrically conductive electrodes, 104 and 105, are extending. The U-shaped electrodes 104 and 105 have first legs, respectively 141 and 151, that extend outside the housing 103 in such a way that the wet garment detector 101 can be pierced onto soft tissue such as the material used to produce incontinence garment, like diapers, undergarment, etc. The U-shaped electrodes 104 and 105 further have second legs, respectively 142 and 152, that extend inside the housing 103 in order to provide connectivity to the electronic circuitry of the wetness sensor and wireless transmitter contained therein.

The wet garment detector 101 of Fig. 1 is able to detect a certain, pre-selected wetness level or saturation degree of the incontinence garment whereon it is pierced, and automatically signals the detection thereof to a receiver. The latter receiver may be a patient terminal fixedly mounted in the patient's room. This patient terminal may further be integrated with a patient file maintenance system, an alarm generating or assistant call system, a building automation system, etc. As an alternative to the fixedly mounted patient terminal, the receiver whereto the wet garment detector 101 signals the wetness condition may be a handheld device carried by a relative or care-giver of the incontinent person or child whose garment is being monitored, e.g. a mobile phone, a pager, a Personal Digital Assistant (PDA), a notebook, a dedicated proprietary device, etc. Yet another possibility is that the wetness condition is signalled by the detector 101 to a central server used to monitor a number of patients, residing for instance in a single institute like a hospital, elderly home, service flat, etc.

The housing 103 is made out of a fluid-tight material, and ideally can be washed for reuse without risk for intake of water that can damage the electronics inside. The housing 103 can for instance be a plastic, PVC, rubber, nylon, etc. housing made watertight through an injection-moulding production process.

The electrodes 104 and 105 - or the piercing pins in an alternative embodiment where the electrodes are not used for piercing the wet garment detector onto the garment - are made out of non-corrosive material. To minimize the risk for injuries, the electrodes (or piercing pins) are made flat (instead of round) and are U-shaped such that the opening ends of the electrodes (or piercing pins) face downwards and the piecing direction becomes a downward movement of the detector. The U-shaped electrodes (or piercing pins) further have the advantage that the location where the wetness condition is sensed, i.e. between the opening ends of the electrodes, remains below the location where the electrodes (or piercing pins) are pierced into the exterior surface of the incontinence garment. The latter exterior surface typically is a plastic, watertight surface. The location where the detector is pierced through the exterior surface will leak when it becomes wet, so it is important that the wetness condition that is signalled is measured below the point where the detector is pierced into the garment. The electrodes 104 and 105 (or piercing pins) may for instance be made out of stainless steel or phosphor copper. Where the electrodes 104 and 105 (or piercing pins) enter the housing 103 of the detector 101, the latter housing 103 may be made watertight through the use of silicon. Where the electrodes are soldered or connected to the electronics of the wetness sensor, measures may be taken to avoid corrosion and to minimize the mechanical forces transferred onto the electronic components and/or prints of the sensor as a result of moving or piercing the electrodes 104 and 105 into the garment tissue. The length of the legs of the U-shaped electrodes 104 and 105 may range for instance from 5 mm (millimeters) up to 3 cm (centimeters). When piercing the detector 101 onto the incontinence garment, care must be taken that the electrodes (which are supposed to serve as piercing pins) do not enter and leave the diaper. Should this be the case, then the wetness level or saturation degree that is sensed will differ from the selected one because the detector 101 will no longer detect the presence of fluid between the opening ends of the electrodes 104 and 105 but will detect the presence of fluid between the sections of the electrodes 104 and 105 that are within the garment. Typically, signalling shall then occur at a wetness level or saturation degree that is higher than the one expected, leaving the care-giver less time to change the diaper or garment before complete saturation and/or leakage.

The detector 101 includes a wetness sensor 201 that permanently monitors the wetness of the garment. This wetness sensor 201 operates according to well-known principles. Examples of known wetness sensors are resistive-based, capacitive-based, electrochemical-based, chemical-based wetness sensors. All these types of sensors may be incorporated in different embodiments of the detector according to the present invention. The sensor 201, depicted in Fig. 2, that forms part of the detector 101 drawn in Fig. 1 does not generate a signal, i.e. an electric current, as long as the pre-selected wetness condition is not reached but immediately generates a signal when the pre-selected wetness level has been achieved inside the diaper or incontinence garment. The sensor 201 is resistive based. The two electrodes 104 and 105 that are pierced into the incontinence garment are short-circuited when the fluids reach the open ends of these electrodes 104 and 105, as a result of which the electronic circuitry of the wetness sensor 201 starts to conduct and activates the wireless transmitter 205 or TX. Indeed, as a result of the short-circuiting, the electrodes 104 and 105 become conductive and the switch transistor 204 is controlled to close. Through the closing of transistor 204, the second pole of the battery or power supply 206 becomes connected to the second power supply terminal of transmitter 205 and the transmitter is activated. The first pole of the battery or power supply 206 may be assumed to be permanently connected to the first power supply terminal of the transmitter 205. The resistors 203 or R1 and 202 or R2 may for instance be designed to equal respectively 10 kΩ (kilo-ohms) and 470 kΩ and have as task to reduce the current and consequently the power consumption when the electrodes 104 and 105 become short-circuited through the fluids in the diaper. Transistor T1 can be the general purpose npn transistor BC847c from manufacturer Zowie. The transmitter 205 is a wireless transmitter, but alternative embodiments may be deployed with wired transmitters and receivers. The wireless transmitter can be based on standard technology such as Bluetooth or WiFi, or implement a proprietary signalling protocol for conveying the wetness condition signal from the transmitter to the receiver such as for instance the Easywave RF transmitter from manufacturer Niko. The wetness condition signal may be a logic 0/1 signal having two states, i.e. a state indicative for a dry condition and a state indicative for the wet condition, but it may alternatively be a more sophisticated signalling wherein it is possible to communicate the exact wetness level or saturation degree, or at least give a gradual indication of the saturation of the incontinence garment. The power supply 206 may for instance be a 3V rechargeable battery.

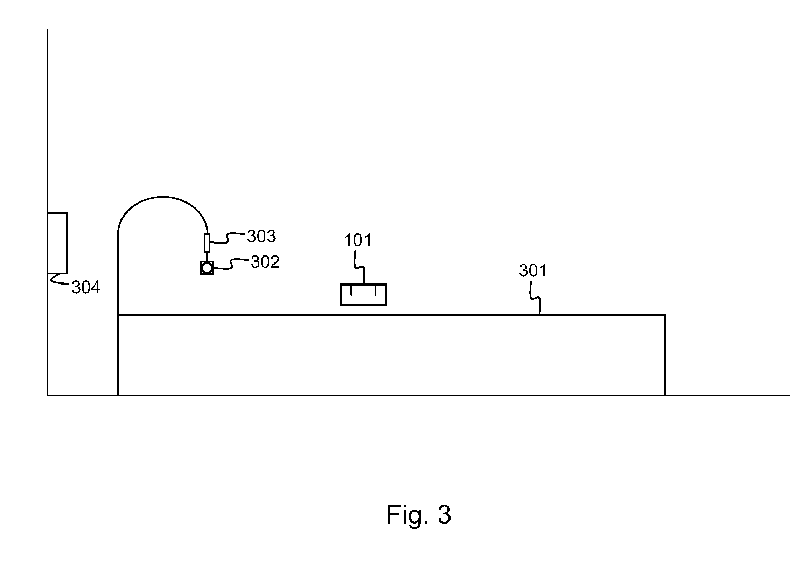

Fig. 3 shows a possible setup in a hospital room where the wet garment detector 101 according to the present invention is used. The wet garment detector 101 that is attachably pierced onto the diaper of an incontinent patient communicates wirelessly with a wall-mounted patient terminal 304 in the patient's room. The patient terminal 304 further receives alarm signals from an alarm button 302 on or near the patient's bed 301. The alarm button 302 also communicates wirelessly with the terminal 304. In Fig. 3, the alarm button 302 is mounted above the patient's bed using a breakable pull relief 303.

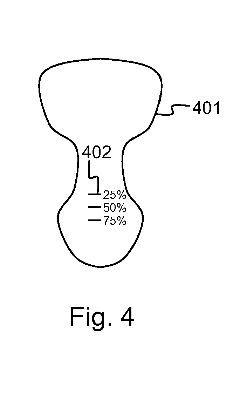

Fig. 4 at last shows a diaper 401 which advantageously is used in conjunction with the wet garment detector 101 according to the present invention. The diaper 401 contains on its exterior surface markings 402 indicative for the location where the detector 101 is to be pierced onto the diaper 401 in case a predefined wetness degree must be signalled. The markings 402 may be labelled as is indicated in Fig. 4, although the location in the diaper where an exact saturation degree or wetness level is reached, e.g. the 25% level, 50 % level or 75 % level, may depend on the person wearing the diaper, the gender of that person, and other factors, such that the labels may be inaccurate or at least should interpreted taking into account personal experience. Although optional, the markings 402 on the exterior surface of diaper 401 provide a handy way for users of the wet garment detector according to the present invention to determine the location where the detector shall be pierced onto the diaper in order to signal a pre-selected wetness condition.

It is remarked that although the invention has been described through an example detector whose piercing pins correspond to electrodes of a wetness sensor, this aspect is only optional and any skilled person shall appreciate that variant embodiments of the piercable detector according to the present invention can be conceived without using the electrodes as piercing pins. Advantageously, the piercing pins (or electrodes) of the detector according to the present invention are flat pins to reduce the risk for injuries should the open ends of the pins get in touch with the patient's skin.

The presence of a transmitter in the here above described embodiment of the invention, is entirely optional, since the wetness condition may alternatively be signalled by visual/audible elements such a s a LED, loudspeaker, buzzer, ringer, etc. Visual indicators however have the drawback that the patient still may have to be uncovered regularly while sleeping in order to check the wetness status of the diaper. Audible indicators have as drawback that the care-giver or relative that is supposed to change the diaper needs to stay within the vicinity of the patient.

Another remark is that the detector according to the present invention may be integrated in a system which also measures or monitors other parameters such as the heart pulse, blood pressure, falling detection, etc.

Although the present invention has been illustrated by reference to specific embodiments, it will be apparent to those skilled in the art that the invention is not limited to the details of the foregoing illustrative embodiments, and that the present invention may be embodied with various changes and modifications without departing from the spirit and scope thereof. The present embodiments are therefore to be considered in all respects as illustrative and not restrictive, the scope of the invention being indicated by the appended claims rather than by the foregoing description, and all changes which come within the meaning and range of equivalency of the claims are therefore intended to be embraced therein. In other words, it is contemplated to cover any and all modifications, variations or equivalents that fall within the spirit and scope of the basic underlying principles and whose essential attributes are claimed in this patent application. It will furthermore be understood by the reader of this patent application that the words "comprising" or "comprise" do not exclude other elements or steps, that the words "a" or "an" do not exclude a plurality, and that a single element, such as a computer system, a processor, or another integrated unit may fulfil the functions of several means recited in the claims. Any reference signs in the claims shall not be construed as limiting the respective claims concerned. The terms "first", "second", third", "a", "b", "c", and the like, when used in the description or in the claims are introduced to distinguish between similar elements or steps and are not necessarily describing a sequential or chronological order. Similarly, the terms "top", "bottom", "over", "under", and the like are introduced for descriptive purposes and not necessarily to denote relative positions. It is to be understood that the terms so used are interchangeable under appropriate circumstances and embodiments of the invention are capable of operating according to the present invention in other sequences, or in orientations different from the one(s) described or illustrated above.

## Claims

1. A wet garment detector (101) for detecting a wetness condition of garment comprising:
a housing (103) for containing electronic components; and
a wetness sensor (201) at least partially contained in said housing (103),
**CHARACTERIZED IN THAT** said wet garment detector (101) further comprises means (104, 105) for piercing said detector (101) onto said garment to thereby removably attach said detector (101) to said garment.

2. A wet garment detector (101) according to claim 1,
**CHARACTERIZED IN THAT** said means (104, 105) for piercing comprise two detection electrodes that form part of said wetness sensor (201).

3. A wet garment detector (101) according to claim 2,
**CHARACTERIZED IN THAT** said detection electrodes (104, 105) are composed of non-corroding material.

4. A wet garment detector (101) according to claim 2,
**CHARACTERISED IN THAT** said detection electrodes (104, 105) are U-shaped, one leg (141, 151) of each U-shaped detection electrode (104, 105) extending outside said housing (103) for piercing into said garment and a second leg (142, 152) of each U-shaped detection electrode (104, 105) extending inside said housing (103) for connectivity to electronic components of said wetness sensor (201 ).

5. A wet garment detector (101 ) according to claim 1,
**CHARACTERIZED IN THAT** said wetness sensor (201) comprises:
two detection electrodes (104, 105) adapted to be non-conductive in a first state wherein said wetness condition is not detected, and to be conductive in a second state wherein said wetness condition is detected;
a power supply, having a first terminal permanently connected to a first power supply terminal of an active element (205) able to signal said wetness condition, and having a second terminal;
a switch (204) whose control terminal is connected to a detection electrode (105) of said detection electrodes (104, 105), and which is adapted to connect said second terminal of said power supply to a second power supply terminal of said active element (205) when said detection electrodes (104, 105) become conductive.

6. A wet garment detector (101) according to claim 1,
**CHARACTERIZED IN THAT** said detector (101) further comprises means for visibly or audibly signalling said wetness condition of said garment.

7. A wet garment detector (101) according to claim 1,
**CHARACTERIZED IN THAT** said detector (101) further comprises a transmitter (205) for wired or wireless transmission of a wetness signal to a receiver (304).

8. A wet garment detector (101) according to claim 7,
**CHARACTERIZED IN THAT** said receiver (304) is further adapted to receive signalling from one or more of the following:
an alarm generating device (302);
a medical sensor.

9. A wet garment detector (101) according to claim 1,
**CHARACTERIZED IN THAT** said means for piercing (104, 105) are adapted to be pierced at different locations into said garment, to thereby enable selection of said wetness condition of said garment detectable by said detector (101).

10. A method for detecting a wetness condition of garment comprising removably attaching a wet garment detector (101) having a housing (103) and wetness sensor (201) at least partially contained in said housing (103) to said garment,
**CHARACTERIZED IN THAT** said step of removably attaching said wet garment detector (101) comprises piercing said wet garment detector (101) onto said garment.

11. A method according to claim 10,
**CHARACTERIZED IN THAT** said method further comprises determining a location onto said garment thereby selecting said wetness condition of said garment detectable by said detector (101), and piercing said wet garment detector (101) at said location onto said garment.

## Amended claims

### Amended claims in accordance with Rule 137(2) EPC.

**1.** A wet garment detector (101) for detecting a wetness condition of garment comprising:
a housing (103) for containing electronic components; and
a wetness sensor (201) at least partially contained in said housing (103),
**CHARACTERIZED IN THAT** said wet garment detector (101) further comprises means (104, 105) for piercing said detector (101) into the exterior surface of said garment such that the open ends of said means (104, 105) for piercing remain within said garment to thereby removably attach said detector (101) to said garment.

**2.** A wet garment detector (101) according to claim 1,
**CHARACTERIZED IN THAT** said means (104, 105) for piercing comprise two detection electrodes that form part of said wetness sensor (201).

**3.** A wet garment detector (101) according to claim 2,
**CHARACTERIZED IN THAT** said detection electrodes (104, 105) are composed of non-corroding material.

**4.** A wet garment detector (101) according to claim 2,
**CHARACTERISED IN THAT** said detection electrodes (104, 105) are U-shaped, one leg (141, 151) of each U-shaped detection electrode (104, 105) extending outside said housing (103) for piercing into said garment and a second leg (142, 152) of each U-straped detection electrode (104, 105) extending inside said housing (103) for connectivity to electronic components of said wetness sensor (201).

**5.** A wet garment detector (101) according to claim 1,
**CHARACTERIZED IN THAT** said wetness sensor (201) comprises:
two detection electrodes (104, 105) adapted to be non-conductive in a first state wherein said wetness condition is not detected, and to be conductive in a second state wherein said wetness condition is detected;
a power supply, having a first terminal permanently connected to a first power supply terminal of an active element (205) able to signal said wetness condition, and having a second terminal;
a switch (204) whose control terminal is connected to a detection electrode (105) of said detection electrodes (104, 105), and which is adapted to connect said second terminal of said power supply to a second power supply terminal of said active element (205) when said detection electrodes (104, 105) become conductive.

**6.** A wet garment detector (101) according to claim 1,
**CHARACTERIZED IN THAT** said detector (101) further comprises means for visibly or audibly signalling said wetness condition of said garment.

**7.** A wet garment detector (101) according to claim 1,
**CHARACTERIZED IN THAT** said detector (101) further comprises a transmitter (205) for wired or wireless transmission of a wetness signal to a receiver (304).

**8.** A wet garment detector (101) according to claim 7,
**CHARACTERIZED IN THAT** said receiver (304) is further adapted to receive signalling from one or more of the following:
an alarm generating device (302);
a medical sensor.

**9.** A wet garment detector (101) according to claim 1,
**CHARACTERIZED IN THAT** said means for piercing (104, 105) are adapted to be pierced at different locations into said garment, to thereby enable selection of said wetness condition of said garment detectable by said detector (101).

**10.** A method for detecting a wetness condition of garment comprising removably attaching a wet garment detector (101) having a housing (103) and wetness sensor (201) at least partially contained in said housing (103) to said garment,
**CHARACTERIZED IN THAT** said step of removably attaching said wet garment detector (101) comprises piercing said wet garment detector (101) into the exterior surface of said garment such that the open ends of piercing means (104, 105) remain within said garment.

**11.** A method according to claim 10,
**CHARACTERIZED IN THAT** said method further comprises determining a location onto said garment thereby selecting said wetness condition of said garment detectable by said detector (101), and piercing said wet garment detector (101) at said location onto said garment.
